(19) Europäisches Patentamt
European Patent Office
Office européen des brevets

(11) Publication number: 0 033 752 B1

# (12) EUROPEAN PATENT SPECIFICATION

(45) Date of publication of patent specification: 08.04.87

(51) Int. Cl.$^4$: C 07 D 263/12

(21) Application number: 80100703.0

(22) Date of filing: 12.02.80

## (54) Preparation of 2-oxazolines by cyclodehydration process employing inorganic iron salts as catalysts.

(43) Date of publication of application:
19.08.81 Bulletin 81/33

(45) Publication of the grant of the patent:
08.04.87 Bulletin 87/15

(84) Designated Contracting States:
BE DE FR GB IT NL

(56) References cited:
FR-A-2 298 559
US-A-2 844 589
US-A-3 681 329
US-A-3 681 333
US-A-3 741 961

(73) Proprietor: THE DOW CHEMICAL COMPANY
2030 Dow Center Abbott Road P.O. Box 1967
Midland, MI 48640 (US)

(72) Inventor: Kaiser, Mark Edward
2808 Blairmont
Midland, MI (US)

(74) Representative: Casalonga, Alain et al
BUREAU D.A. CASALONGA OFFICE JOSSE & PETIT Morassistrasse 8
D-8000 München 5 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 033 752 B1

Courier Press, Leamington Spa, England.

**Description**

This invention pertains to a novel cyclodehydration reaction which produces 2-oxazolines using soluble iron-containing compounds as the catalyst, where the term "soluble" is defined in the following description.

The 2-oxazolines are a known class of compounds whose chemistry has been summarized by Wiley et al., *Chemical Reviews,* Vol. 44, 447—476 (1949); Seeliger et al., *Angew. Chem. International Edition,* Vol. 5, No. 10, 875—888 (1966), and Frump, *Chemical Reviews,* 1971, Vol. 71, No. 5, 483—505.

The 2-oxazolines correspond to the general formula

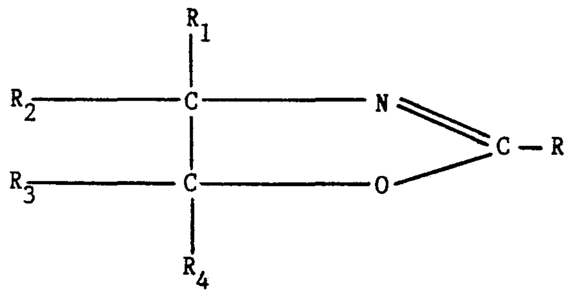

I

wherein R is hydrogen, hydrocarbyl or inertly-substituted hydrocarbyl, and $R_1$—$R_4$ are each independently hydrogen, hydrocarbyl, or an inertly-substituted hydrocarbyl group. The best known oxazolines are those in which R is hydrogen, alkyl or phenyl and $R_1$ and $R_2$ are hydrogen, lower alkyl or hydroxymethyl or derivatives thereof (e.g., esters) and $R_3$ and $R_4$ are each hydrogen.

2-H-2-Oxazoline is the first member of the oxazoline series and corresponds to formula II.

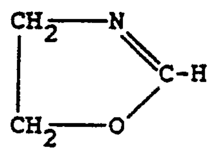

II.

The 2-H-2-oxazolines and particularly II above are generally quite different from the corresponding 2-substituted-2-oxazolines. This is illustrated by the fact that many conventional processes for preparing 2-substituted-2-oxazolines are not particularly satisfactory for the preparation of the corresponding 2-H-2-oxazolines. For example, many 2-substituted-2-oxazolines are conventionally prepared by dehydrochlorinating an N-(β-chloroalkyl)carboxamide with aqueous alkali. Compound II, on the other hand, is produced by this process in only very low yields and is accompanied by decomposition of the desired product.' See H. Wenker, *J. Am. Chem. Soc., 60,* 2152 (1938).

Another common technique for preparing 2-oxazolines is the cyclodehydration of N-(β-hydroxyalkyl)-carboxamides over various catalysts. Litt et al. (U.S. Patents 3,681,329 and 3,681,333, both issued August 1, 1972) claimed that 2-H-2-oxazolines could be prepared by contacting the appropriate formamides with compounds of manganese, cobalt, molybdenum, tungsten and the rare earth metals. Unfortunately, there is no experimental data in Litt et al. which would substantiate this allegation. Hess teaches in U.S. Patent 2,844,589, issued July 22, 1958 that II can be prepared by a cyclodehydration of N-(2-hydroxyethyl)-formamide in the presence of a dehydrating agent (specifically diatomaceous earth, sulfuric acid, aluminum oxide and iron oxide). This Hess citation is considered to be the most pertinent prior art. The yields reported by Hess were higher than the yield reported by Wenker but were still rather low and commercially unsatisfactory. In contrast to this, Litt et al. teach in U.S. Patent 3,562,263, issued February 9, 1971, that 2-substituted-2-oxazolines are prepared in excellent yield by cyclodehydration of N-(2-hydroxyalkyl)carboxamides over aluminum oxide.

FR.A. 2.298.559 (EXXON) describes a process for preparing oxazoline derivatives by reacting a 2,2-disubstituted 2-amino-1-alkanol containing 2 or 3 hydroxyl groups with a hydrocarbyl dicarboxylic acid. The disubstituted material react easily and the use of the catalyst is optional. According to this reference the reaction will not work for unsubstituted or monosubstituted oxazolines and inorganic iron compounds are not suitable.

US—A—3.741.961 (J. E. KMIECIK) describes the cyclisation of organic nitriles with 1, 2- or 1, 3- amino alcohols or aminoothiols in the presence of metal catalysts to prepare 2-oxazoline. This reaction differs totally from that used according to invention.

In view of these differences between 2-H-2-oxazolines and 2-substituted-2-oxazolines in the various methods of preparation, it was surprising to find a class of compounds which would catalyze the cyclohydration of N-(2-hydroxyethyl)formamides and carboxamides to form the corresponding 2-H-2-oxazolines or the 2-substituted-2-oxazolines.

The present invention is a cyclodehydration process for preparing a 2-oxazoline of general formula

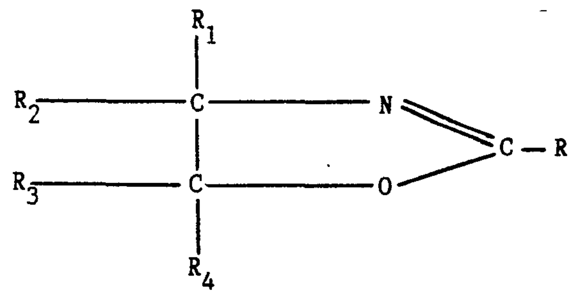

comprising the step of reacting in liquid phase a N-(2-hydroxyalkyl) carboxamide or formamide of general formula

$$R-\overset{\overset{\displaystyle O}{\|}}{C}-NH-CR_1R_2-CR_3R_4-OH \quad \text{(III)}$$

or a carboxylic acid/amide salt precursor thereof wherein R is hydrogen, alkyl of 1 to 17 carbon atoms or phenyl non substituted or substituted by substituents which are inert during the reaction and $R_1$, $R_2$, $R_3$, $R_4$ are independently hydrogen, lower alkyl ($C_1$—$C_6$) non substituted or substituted by substituents which are inert during the reaction.

The carboxamide or formamide of formula III is reacted with 0.005 to 0.4 mol/mol carboxamide or formamide reactant of an inorganic iron compound having a minimum solubility of 100 ppm at a reaction temperature of 140 to 280°C in the carboxamide or formamide reactant. The inorganic compound is not iron oxide or hydroxide.

The discovery that iron-containing compounds would catalyze the instant cyclodehydration reaction was most surprising for reasons set forth above and particularly in view of the teaching by Litt et al. in U.S. Patents 3,681,329 and 3,681,333, both previously identified, that iron compounds are inferior catalysts in this reaction.

Preferred reactants are those wherein R is hydrogen, alkyl of from 1 to about 17 carbon atoms or phenyl and $R_1$ and $R_2$ are hydrogen, lower alkyl ($C_1$—$C_6$), hydroxymethyl or alkanoyloxymethyl groups (alkyl-C(O)—$OCH_2$—) of up to about 17 carbon atoms and $R_3$ and $R_4$ are hydrogen. Most preferred reactants are those wherein R is hydrogen, methyl, ethyl or phenyl, and $R_1$—$R_4$ are each hydrogen. These preferences are based upon the commercial availability of the ethanolamines which are used in preparing such carboxamides.

The N-(2-hydroxyalkyl)carboxamides are conveniently and typically prepared by reacting a carboxylic acid or a lower alkyl ester of a carboxylic acid with an ethanolamine of the formula

$$NH_2-CR_1R_2-CR_3R_4-OH \quad \text{IV.}$$

wherein $R_1$—$R_4$ have the meaning described above.

The carboxylic acid/amine salt which is formed initially in these reactions can be used in the instant process in place of the carboxamide. When such carboxylic acid/amine salts are used, the carboxamide is generated in situ.

The catalysts in the instant cyclodehydration reaction are iron-containing compounds. Suitable catalysts are inorganic iron compounds which are soluble in the carboxamide reactant or liquid reaction medium. The term "soluble" means that the iron-containing compound has at least a minimum solubility (e.g., about 100 parts per million or more) at the reaction temperature. Examples of suitable inorganic iron-containing compounds which can be used as catalysts include: ferrous chloride, ferric chloride, ferric bromide, ferric iodide, ferrous sulfate, ferric sulfate, ferric ammonium sulfate, and ferric potassium sulfate. The iron chlorides, bromides, iodides and sulfate are preferred. Ferrous and ferric chlorides and sulfates are the most preferred catalysts.

The inorganic iron compounds are used in the instant process in small but catalytic amounts and are added in amounts of 0.005 to 0.4 mole of inorganic iron compound per mole of carboxamide or formamide reactant but more or less of the iron-containing compounds can be used, if desired.

The instant cyclodehydration reaction may be conducted neat or in solution with a suitable inert solvent. By "inert" is again meant inert in the process. Suitable such inert solvents include, for example, chlorinated hydrocarbon solvents, aromatic hydrocarbon solvents, cycloaliphatic hydrocarbon solvent and aliphatic hydrocarbon solvents. It is preferred, however, to conduct the reaction without any solvent added (i.e., neat).

The reaction temperature must, obviously, be sufficient to promote the cyclodehydration reaction and is in the range of 140°C to 280°C. Preferred reaction rates have been observed at temperatures of 160°C to 250°C.

The instant cyclodehydration reaction is also preferably conducted under reduced pressure. By conducting the reaction in this manner, product recovery is facilitated in that frequently a reaction temperature may be chosen which is above the boiling point of the 2-oxazoline product and below the boiling point of the N-(2-hydroxyalkyl)carboxamide reactant. In this manner, the 2-oxazoline can be removed from the reaction mixture as a volatile gas essentially as fast as it is formed. This is very desirable since the instant cyclodehydration reaction is a reversible process and by removing the products, the reaction is forced to completion by substantially reducing the reverse reaction. Water normally codistills with the 2-oxazoline product.

The instant process may be conducted in a batch process or in a continuous manner. In the preferred continuous process the N-(2-hydroxyalkyl)carboxamide reactant is metered into the reaction vessel at essentially the same rate as the 2-oxazoline and water are removed overhead as volatile gases.

Example 1

Preparation of 2-H-2-Oxazoline

N-(2-Hydroxyethyl)formamide (100.2 g/1.125 mole) and ferrous chloride tetrahydrate (1.58 g; 0.008 mole) was added to a reaction vessel equipped with a stirring means, heating means, and a distillation means comprising a distillation column with a takeoff head and receiver, and a vacuum source. The pressure over the reaction mixture was adjusted to 50 mm Hg and the temperature raised to 180°C—187°C. The head temperature of the distillate was between about 37°C and about 42°C. The material collected from the overhead distillate was a water white liquid weighing 89.5 g. Analysis of this water white liquid using gas chromatography indicated that it was 79.2 weight percent oxazoline, and a Karl Fischer analysis indicated that the material was 19.9 weight percent water. The yield derived from this analytical data indicated that 2-H-2-oxazoline was produced in 88.7 percent of theoretical yield, based on the N-(2-hydroxyalkyl)formamide charged to the reaction vessel.

The 2-H-2-oxazoline can be recovered from the aqueous distillate, if desired, by solvent extraction using chloroform or other inert water-immiscible organic solvents.

Example 2

2-H-2-Oxazoline was prepared in approximately 84 percent yield following the procedure of Example 1, except for using $FeSO_4 \cdot 7H_2O$ as the catalyst.

Example 3

Similar to Example 1, 2-H-2-oxazoline was prepared in approximately 82 percent yield using hydrated ferric sulfate in place of ferrous chloride as the catalyst.

By way of comparison, it is noted that Hess in U.S. Patent 2,844,589 produced 2-H-2-oxazoline in extremely low yields using ferric oxide as the catalyst. We observed similar poor yields in experiments using ferric hydroxide and ferrous gluconate as catalysts in attempts to prepare 2-H-2-oxazoline. These iron compounds were insoluble in the N-(2-hydroxyethyl)formamide reactant under conditions set forth in the above examples.

Example 4

Using the apparatus described in Example 1, an aliquot of N-(2-hydroxyethyl)propionamide amounting to approximately 0.3 mole and ferric chloride (about 0.05 mole) were charged to the reaction vessel and the pressure reduced to 120 mm Hg. The reaction mixture was then heated to approximately 200°C and held at this reaction temperature until material began to distill overhead. At this point, additional N-(2-hydroxyethyl)propionamide was added dropwise at approximately 1.4 g/minute. During this addition, the pot temperature was maintained at approximately 200°C and the water white distillate came overhead at a temperature of from 65°C to about 72°C. After the addition was complete, the pot temperature was raised to approximately 250°C to drive off residual amounts of the 2-ethyl-2-oxazoline. The distillation head temperature reached a maximum of 90°C in this postheating period. The distillate was a water white liquid upon cooling which was analyzed by gas chromatography and Karl Fischer titration. This analytical data showed that 2-ethyl-2-oxazoline was thus produced in 90 percent yield, based upon the amount of N-(2-hydroxyethyl)propionamide charged to the reaction vessel.

Example 5

Using the procedure set forth in Example 4, 2-ethyl-2-oxazoline was produced in 90.2 percent yield using ferrous chloride tetrahydrate in place of the ferric chloride catalysts.

Example 6

2-Ethyl-2-oxazoline was produced in approximately 76 percent yield in a batch process by warming the salt of propionic acid and ethanolamine over ferrous chloride tetrahydrate (approximately 2 mole percent) to approximately 200°C/50 mm Hg. There was a pause in the rise in temperature during which the acid/

amine salt was converted to the amide. Otherwise, the reaction proceeded essentially the same as Example 5 above. The product was similarly recovered as an overhead distillate with water.

Example 7

In a similar batch process, 2-ethyl-5-methyl-2-oxazoline was produced in approximately 84 percent yield by cyclodehydrating N-(2-hydroxypropyl)propionamide over ferrous chloride tetrahydrate. The reaction proceeded very rapidly under somewhat milder conditions (165°C).

**Claims**

1. A cyclodehydration process for preparing a 2-oxazoline of general formula

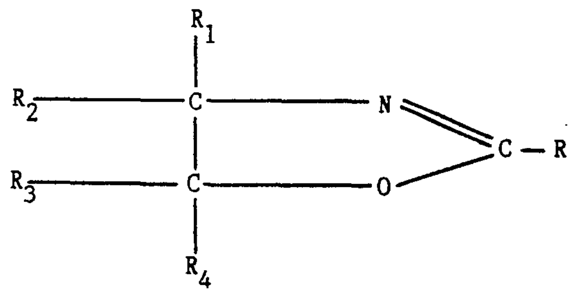

comprising the step of reacting in liquid phase a N-(2-hydroxyalkyl) carboxamide or formamide of general formula

$$R-\overset{O}{\overset{\|}{C}}-NH-CR_1R_2-CR_3R_4-OH \qquad (III)$$

or a carboxylic acid/amine salt precursor thereof wherein R is hydrogen, alkyl of 1 to 17 carbon atoms or phenyl non substituted or substituted by substituents which are inert during the reaction and $R_1$, $R_2$, $R_3$, $R_4$ are independently hydrogen, lower alkyl ($C_1$—$C_6$) non substituted or substituted by substituents which are inert during the reaction, the process being characterized by reacting the carboxamide or formamide of formula III with 0.005 to 0.4 mol/mol carboxamide or formamide reactant of an inorganic iron compound having a minimum solubility of 100 ppm at a reaction temperature of 140 to 280°C in the carboxamide or formamide reactant with the proviso that the inorganic iron compound is not iron oxide or hydroxide.

2. A process as in claim 1 wherein said N-(2-hydroxyalkyl)carboxamide or formamide has the formula III, wherein R is hydrogen, alkyl of from 1 to 17 carbon atoms or phenyl, $R_1$ and $R_2$ are each independently hydrogen, lower alkyl $C_1$—$C_6$, hydroxyméthyl or alkanoyloxymethyl groups of up to 17 carbon atoms, and $R_3$, $R_4$ are each hydrogen.

3. The process of claim 2 and further characterized in that R is methyl, ethyl or phenyl and $R_1$—$R_4$ are each hydrogen.

**Patentansprüche**

1. Ein Cyclodehydratisierungsverfahren zur Herstellung eines 2-Oxazolin der allgemeinen Formel

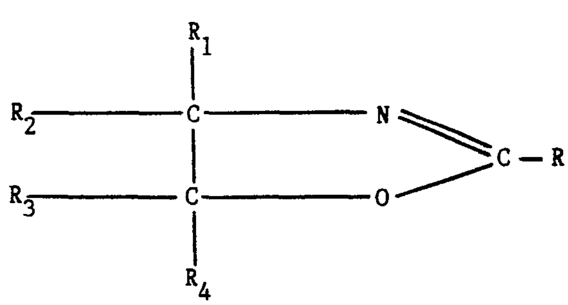

I

beinhaltend den Schritt einer Reaktion eines N-(2-hydroxyalkyl)carboxamid bzw. -formamid der folgenden allgemeinen Formel in flüssiger Phase:

$$R-\overset{\overset{O}{\|}}{C}-NH-CR_1R_2-CR_3R_4-OH \qquad (III)$$

bzw. eines Carbonsäure/Aminsalzes als Vorläufer desselben, ind er R Wasserstoff, ein Alkyl mit 1 bis 17 Kohlenstoffatomen oder ein nichtsubstituiertes oder mit bei der Reaktion inerten Substituenten substituiertes Phenyl ist, und $R_1$, $R_2$, $R_3$, $R_4$ unabhängig voneinander Wasserstoff, ein niederes ($C_1$—$C_6$) nichtsubstituiertes oder mit bei der Reaktion inerten Substituenten substituiertes Alkyl sind, wobei das Verfahren dadurch gekennzeichnet ist, daß das Carboxamid bzw. Formamid der Formel III mit einem 0,005 bis 0,4 mol/mol Carboxamid- oder Formaid-Reaktionsteilnehmer einer anorganischen Eisenverbindung mit einer Mindestlöslichkeit von 100 ppm bei einer Reaktionstemperatur von 140 bis 280°C im Carboxamid- oder Formamid-Reaktionsteilnehmer zur Reaktion gebracht wird, unter der Bedingung, daß es sich bei der Eisenverbindung weder um Eisenoxid noch -hydroxid handelt.

2. Ein Verfahren gemäß Anspruch 1, in dem das genannte N-(2-hydroxyalkyl)carboxamid bzw. -formamid der Formel III entspricht, in der R gleich Wasserstoff, ein Alkyl mit 1 bis 17 Kohlenstoffatomen oder Phenyl ist, $R_1$ und $R_2$ jeweils unabhängig voneinander Wasserstoff, ein niederes Alkyl $C_1$—$C_6$, Hydroxymethyl- oder Alkanoyloxymethylgruppen mit bis zu 17 Kohlenstoffatomen sind, und $R_3$, $R_4$ jeweils Wasserstoff ist.

3. Das Verfahren gemäß Anspruch 2, weiter dadurch gekennzeichnet, daß R gleich methyl, Ethyl oder Phenyl, und $R_1$—$R_4$ jeweils Wasserstoff sind.

**Revendications**

1. Procédé de cyclisation-déshydratation, pour la préparation d'une 2-oxazoline de formule générale:

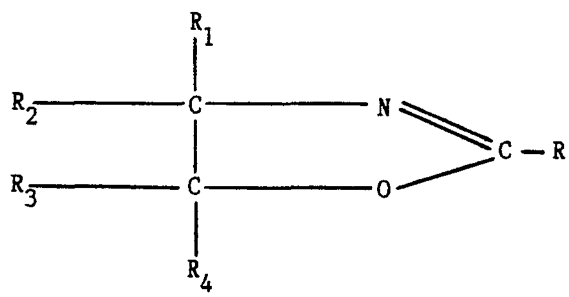

comprenant l'étape consistant à faire réagir en phase liquide un N-(1-hydroxyalkyl)-carboxamide ou -formamide, de formule générale:

$$R-\overset{\overset{O}{\|}}{C}-NH-CR_1R_2-CR_3R_4-OH \qquad (III)$$

ou son précurseur, sel d'amine et d'acide carboxylique, dans lequel R est un atome d'hydrogène ou un groupe alkyle de 1 à 17 atomes de carbone ou un groupe phényle non substitué ou substitué par des substituants qui sont inertes au cours de la réaction, et $R_1$, $R_2$, $R_3$ et $R_4$ sont, indépendamment, des atomes d'hydrogène, ou des groupes alkyle inférieur ($C_1$—$C_6$) non substitués ou substitués par des substituants qui sont inertes au cours de la réaction, le procédé étant caractérisé enc e que l'on fait réagir le carboxamide ou formamide de formule III avec 0,005 à 0,4 mole, par mole du réactif carboxamide ou formamide, d'un composé minéral du fer présentant une solubilité minimale de 100 ppm, à une température de réaction de 140—280°C, dans le réactif carboxamide ou formamide, à condition que le composé minéral du fer ne soit pas un oxyde ou un hydroxyde du fer.

2. Procédé conforme à la revendication 1, dans lequel ledit N-(2-hydroxyalkyl)-carboxamide ou -formamide présente la formule III dans laquelle R est une atome d'hydrogène ou un groupe alkyle de 1 à 17 atomes de carbone ou un groupe phényle, $R_1$ et $R_2$ sont chacun, indépendamment, un atome d'hydrogène ou un groupe alkyle inférieur ($c_1$—$C_6$) hydroxyméthyle ou alcanoyloxyméthyle pouvant comporter jusqu'à 17 atomes de carbone, et $R_3$ et $R_4$ sont chacun un atome d'hydrogène.

3. Procédé conforme à la revendication 2, caractérisé en outre en ce que R est un groupe méthyle, éthyle, ou phényle, et $R_1$, $R_2$, $R_3$ et $R_4$ sont chacun un atome d'hydrogène.